(19) Europäisches Patentamt
European Patent Office
Office européen des brevets

(11) Veröffentlichungsnummer: **0 138 093**
**A2**

(12) # EUROPÄISCHE PATENTANMELDUNG

(21) Anmeldenummer: **84111214.7**

(22) Anmeldetag: **20.09.84**

(51) Int. Cl.⁴: **C 07 D 217/10**, C 07 D 215/10, C 07 C 121/70 // C08K5/34

(30) Priorität: **30.09.83 DE 3335589**

(43) Veröffentlichungstag der Anmeldung: **24.04.85 Patentblatt 85/17**

(84) Benannte Vertragsstaaten: **AT CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Jonas, Friedrich, Dr., Krugenofen 15, D-5100 Aachen (DE)**
Erfinder: **Hocker, Jürgen, Dr., Eichenweg 6, D-5060 Bergisch-Gladbach 2 (DE)**

(54) **Verfahren zur Herstellung von TCNQ-Komplexen.**

(57) Die Erfindung betrifft ein Verfahren zur Herstellung von 7,7,8,8-Tetracyanochinodimethan enthaltenden Charge-Transfer-Komplexen durch Umsetzung von TCNQ mit organischen Kationiodiden oder durch Umsetzung von stickstoffhaltigen Heteroaromaten und tertiären Aminen mit $H_2TCNQ$, in dem geringere Mengen Lösungsmittel verwendet werden als zur vollständigen Lösung des TCNQ erforderlich sind.

EP 0 138 093 A2

BAYER AKTIENGESELLSCHAFT 5090 Leverkusen, Bayerwerk

Konzernverwaltung RP
Patentabteilung

G/by-c

## Verfahren zur Herstellung von TCNQ-Komplexen

Komplexsalze aus dem 7,7,8,8-Tetracyano-p-chinodimethan-anion (TCNQ),

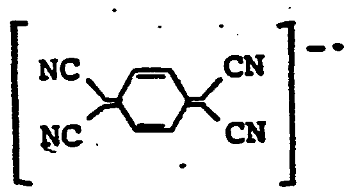

neutralem TCNQ und anorganischen bzw. organischen Kationen sind als elektrisch leitende Verbindungen bekannt und haben in der letzten Zeit großes Interesse, z.B. zur Herstellung von Kondensatoren, gefunden.

Diese Komplexe können durch Umsetzung von TCNQ mit organischen Kationiodiden hergestellt werden [J. Am. Chem. Soc. **84**, 3374-3387 (1962)],

z.B. $4\ TCNQ + 3\ M^+J^- \longrightarrow 2\ M^+ . TCNQ^{\ominus} . TCNQ + m^+J_3^-$

Reaktionsschema I

Hierbei wird ein TCNQ-Molekül durch Iodid zum TCNQ-Anion unter Freisetzung von Iod, das mit überschüssigem Jodid zum $J_3^{\ominus}$-Anion reagiert, reduziert.

Ein anderes Verfahren besteht in der Umsetzung von stickstoffhaltigen Heteroaromaten und tertiären Aminen mit $H_2TCNQ$ (I) und TCNQ, z.B.

3 TCNQ + 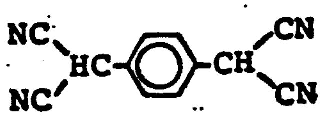 + $2(C_2H_5)_3N \longrightarrow$

(I)

$2(C_2H_5)_3\overset{+}{N}H \; . \; (TCNQ)_2^{-}$

Reaktionsschema II

Bei beiden Verfahren werden in der Regel die Reaktanden in geeigneten Lösungsmitteln meist bei erhöhter Temperatur gelöst und anschließend die Lösungen vereinigt. Als Lösungsmittel wird meistens Acetonitril oder Methylenchlorid verwendet.

Da sich TCNQ auch in der Hitze nur sehr schlecht in den verwendeten Lösungsmitteln löst, (Löslichkeit in Methylenchlorid bei Rückfluß $\approx$ 6 g/l), sind für diese Verfahren große Mengen Lösungsmittel erforderlich, die anschließend aufgearbeitet werden müssen. Herstellung der TCNQ-Komplexsalze mit weniger Lösungsmittel als zum Lösen bei Rückfluß erforderlich ist, führt nicht zu reinen Produkten (Vergleichsbeispiel 1); TCNQ wird

nur unvollständig umgesetzt. Außerdem können die Komplexe durch die lange Reaktionszeit bei hoher Temperatur teilweise zersetzt werden.

Überraschenderweise wurde gefunden, daß die Herstellung der TCNQ-Komplexe unter Verwendung von geringen Mengen Lösungsmitteln möglich ist, wenn man das organische Kationiodid im Lösungsmittel vorlegt und das TCNQ unter Wiederverwendung des Lösungsmittels (z.B. durch Extraktion mit einer Soxhlettapparatur) kontinuierlich zudosiert.

Gegenstand der Erfindung ist daher ein Verfahren zur Herstellung von 7,7,8,8-Tetracyanochinolinmethan enthaltenden Charge-Transfer-Komplexen durch Umsetzung von TCNQ mit organischen Kationiodiden oder durch Umsetzung von stickstoffhaltigen Heteroaromaten und tertiären Aminen mit $H_2$TCNQ und TCNQ, das dadurch gekennzeichnet ist, daß geringere Mengen Lösungsmittel verwendet werden als zur vollständigen Lösung des TCNQ erforderlich sind.

Ein weiterer Vorteil des erfindungsgemäßen Verfahrens liegt darin, daß die TCNQ-Komplexe in Form sehr feiner Nadeln erhalten werden, wie sie vorteilhaft zur antistatischen Ausrüstung von Polymeren verwendet werden (siehe DE 30 05 849), während die bei der üblichen Herstellung anfallenden Komplexe für diesen Verwendungszweck nicht geeignet sind.

Für die Durchführung des erfindungsgemäßen Verfahrens geeignete Lösungsmittel sind organische Lösungsmittel,

wie z.B. Halogenkohlenwasserstoffe, Methylenchlorid, Chloroform, Tetrachlorkohlenstoff, 1,2-Dichlorethan, 1,1,2-Trichlorethan; Acetonitril; Alkohole, wie Methanol, Ethanol, Isopropanol; aliphatische Ketone, wie Aceton, Methylethylketon; acyclische und cyclische Ether, z.B. Diethylether, Tetrahydrofuran. Bevorzugt sind hierbei Lösungsmittel mit niedrigem Siedepunkt (unter 100°C), um eine thermische Schädigung des gebildeten Komplexes zu vermeiden.

Besonders bevorzugte Lösungsmittel haben einen Siedepunkt unter 60°C, z.B. Methylenchlorid.

Bezogen auf eingesetztes TCNQ können sehr geringe Mengen Lösungsmittel verwendet werden. Um eine technische Durchführbarkeit des Verfahrens zu gewährleisten, ist es sinnvoll, mindestens 10 ml Lösungsmittel pro Gramm eingesetztem TCNQ zu verwenden. Die bevorzugte Lösungsmittelmenge liegt zwischen 15 und 30 ml Lösungsmittel pro Gramm eingesetztem TCNQ, kann aber auch darüber liegen.

Erfolgt die Herstellung der CT-Komplexe nach Reaktionsschema II, gilt für die Summe aus eingesetztem TCNQ und $H_2TCNQ$ das oben Gesagte. Für die Durchführung des Verfahrens sind prinzipiell alle organischen Kationiodide bzw. tertiären Amine oder N-Heteroaromaten geeignet.

Ein Überblick findet sich in J. Am. Chem. Soc. **84**, 3370 - 3387 (1962).

Bevorzugt sind solche Kationiodide, die im organischen Lösungsmittel löslich sind, z.B. N-Methylchinoliniumiodid, N-Butylisochinoliniumiodid.

Für die Durchführung des Verfahrens geeignete Apparaturen (z.B. Thielepape-Aufsatz, Soxhlett-Extraktor) sind in Organikum, VEB Verlag der Wissenschaften 1973, S. 66 - 67 beschrieben. Es können aber auch andere Geräte, die eine kontinuierliche Zudosierung des TCNQ unter Wiederverwendung des Lösungsmittels ermöglichen, verwendet werden.

Man geht hierbei so vor, daß das Kationiodid im Lösungsmittel vorgelegt und das TCNQ in den Extraktor eingefüllt wird. Anschließend wird so lange bei Rückfluß gehalten, bis alles TCNQ gelöst ist. Die Isolierung des gebildeten Charge-Transfer-Komplexes kann durch einfaches Absaugen erfolgen. Man erhält die Komplexe in hohen Ausbeuten und hoher Reinheit.

Die so erhaltenen Komplexe können hervorragend zur antistatischen Ausrüstung von Polymeren, z.B. Polyolefinen wie Polyethylen, Polystyrol, Polyisopren; Polyvinylchlorid; Polyamide, wie Polyamid-6,6; Polyester wie Polyethylenterephthalat; Polycarbonaten, Polyacrylnitril, Acrylnitril-Butadien-Styrol-Copolymerisaten (ABS), Polyvinylacetat, Celluloseestern wie Celluloseacetat, Polyurethanen verwendet werden.

## Beispiele

### Vergleichsbeispiel 1

27,0 g N-Methylchinoliniumiodid, 27,2 g TCNQ und 500 ml Methylenchlorid werden 24 Stunden in einer Rührapparatur bei Rückfluß gerührt. Anschließend wird abgesaugt und getrocknet. Das Produkt enthält größere Mengen nicht umgesetztes TCNQ, das als gelbe Kristalle sichtbar ist.

### Vergleichsbeispiel 2

27,0 g N-Methylchinoliniumiodid werden bei Rückfluß in 300 ml Acetonitril gelöst. Zu dieser Lösung wird eine siedende Lösung von 27,2 g TCNQ in 1800 ml Acetonitril gegeben. Der beim Abkühlen ausfallende Komplex wird abgesaugt und getrocknet.

Beispiel 1

18,0 g N-Isopropylisochinoliniumiodid werden in einer Rührapparatur mit Soxhlettaufsatz in 200 ml Methylenchlorid gelöst. In den Soxhlettaufsatz werden 16,3 g TCNQ gegeben, das bei Rückfluß kontinuierlich extrahiert wird. Die Reaktion ist beendet, wenn alles TCNQ herausgelöst ist. Der ausgefallene Komplex wird abgesaugt, mit wenig Methylenchlorid gewaschen und getrocknet. Man erhält 22,3 g ≙ 96 % der Theorie CT-Komplex der Zusammensetzung:

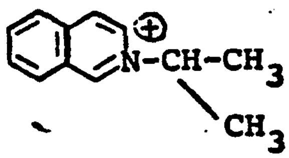 • $(TCNQ)_2^-$

$C_{36}H_{22}N_9$ (580) Ber.: 74,5 % C 3,8 % H 21,7 % N
Gef.: 74,3 % C 4,0 % H 21,7 % N

Beispiel 2

27,6 g N-Octyl-triphenylphosphoniumiodid, 10,2 g TCNQ und 150 ml Methylenchlorid werden, wie in Beispiel 1 beschrieben, zur Reaktion gebracht und aufgearbeitet.

Man erhält 17,6 g ≙ 90 % der Theorie CT-Komplex der Zusammensetzung

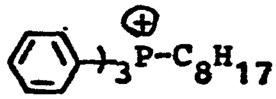 • $(TCNQ)_2^-$

| | | | | |
|---|---|---|---|---|
| $C_{50}H_{40}N_8P$ (783) | Ber.: | 76,6 % C | 5,1 % H | 14,3 % N |
| | Gef.: | 76,2 % C | 4,8 % H | 14,7 % N |

Beispiel 3

18,1 g N-n-Butylisochinoliniumiodid, 16,3 g TCNQ und 200 ml Methylenchlorid werden, wie in Beispiel 1 beschrieben, zur Reaktion gebracht.
Man erhält 21 g ≙ 90 % der Theorie CT-Komplex der Zusammensetzung

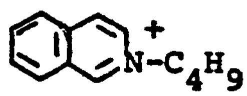 • $(TCNQ)_2^-$

| | | | | |
|---|---|---|---|---|
| $C_{37}H_{24}N_9$ (594) | Ber.: | 74,8 % C | 4,0 % H | 21,2 % N |
| | Gef.: | 74,4 % C | 4,2 % H | 21,2 % N |

Beispiel 4

16,3 g N-Methylchinoliniumiodid, 16,3 g TCNQ und 300 ml Methylenchlorid werden, wie in Beispiel 1 beschrieben, zur Reaktion gebracht.

Man erhält 21,2 g ≙ 96 % der Theorie CT-Komplex der Zusammensetzung

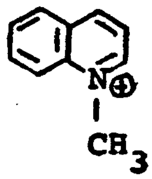 • $(TCNQ)_2^-$

| | | | | | |
|---|---|---|---|---|---|
| $C_{34}H_{18}N_9$ | (552) | Ber.: | 73,9 % C | 3,3 % H | 22,8 % N |
| | | Gef.: | 73,7 % C | 3,9 % H | 22,9 % N |

<u>Beispiel 5</u>

5,2 g Chinolin werden in einer Rührapparatur mit Soxhlettaufsatz in 200 ml Methylenchlorid gelöst. In den Soxhlettaufsatz werden 12,2 g TCNQ und 4,0 g 7,8-Dihydro-TCNQ gegeben und die Reaktion, wie in Beispiel 1 beschrieben, durchgeführt.

Man erhält 19,6 g ≙ 91 % der Theorie CT-Komplex der Zusammensetzung

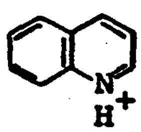 • $(TCNQ)_2^-$

| | | | | | |
|---|---|---|---|---|---|
| $C_{33}H_{16}N_9$ | (538) | Ber.: | 73,6 % C | 3,0 % H | 23,4 % N |
| | | Gef.: | 73,3 % C | 3,2 % H | 23,0 % N |

Anwendungsbeispiel

200 g einer 10 %igen Lösung eines handelsüblichen Polycarbonats der Molmasse 59000 in Methylenchlorid wird mit 0,2 g TCNQ-Komplex nach Beispiel 4 ≙ Folie A bzw. 0,2 g TCNQ-Komplex nach Vergleichsbeispiel 2 ≙ Folie B versetzt und bis zur homogenen Verteilung des Komplexes gerührt. Anschließend wird die Lösung zu einer nach dem Verdunsten des Lösungsmittels ≈ 100 µm dicken Folie gegossen und der Oberflächenwiderstand bestimmt.

Folie A R = $1,4 \cdot 10^{7}\,\Omega \times cm$

Folie B R = $2,3 \cdot 10^{13}\,\Omega \times cm$ (nach DIN 53 482)

Patentanspruch

Verfahren zur Herstellung von 7,7,8,8-Tetracyanochinodimethan enthaltenden Charge-Transfer-Komplexen durch Umsetzung von TCNQ mit organischen Kationiodiden oder durch Umsetzung von stickstoffhaltigen Heteroaromaten und tertiären Aminen mit $H_2$TCNQ und TCNQ, dadurch gekennzeichnet, daß geringere Mengen Lösungsmittel verwendet werden als zur vollständigen Lösung des TCNQ erforderlich sind.